# EUROPEAN PATENT APPLICATION

(11) **EP 1 709 905 A1**
(43) Date of publication of application: **11.10.2006**
(21) Application number: 05007589.4
(22) Date of filing: 06.04.2005
(51) Int. Cl.: A61B 5/113

(54) **Belt system for measuring respiration dependent girth change during magnetic resonance imaging**

(71) Applicant: Deutsches Krebsforschungszentrum Stiftung des öffentlichen Rechts, 69120 Heidelberg (DE)
(72) Inventor: Remmert, Gregor, 69115 Heidelberg (DE); Echner, Gernot, 69257 Wiesenbach (DE)
(74) Representative: Ganahl, Bernhard

(57) **Abstract**

The invention relates to a respiratory belt system for being used in a magnetic resonance imaging environment.

This system comprises a belt being elastically extendable, an optical transducer and an optical pattern being coupled to the belt. During extension or contraction of the belt the optical pattern is moved relative to the light source for a distance that is substantially identical to the length of the extension or the contraction of the belt. The sensor creates a measurement signal corresponding to the change in length of the belt.

## Description

The invention relates to a respiratory belt system.

Respiratory belt systems comprise a belt being wrapped around a person to be examined, a sensor unit for outputting a sensor signal corresponding to changes of the girth of the person to be examined and an evaluation unit for evaluating the sensor signals.

When obtaining a tomogram using magnetic resonance imaging (MRI or NMR) techniques it is known to monitor the respiratory motion of the examination subject during certain types of exposures, so that the generation of the image can be gated, synchronized with the respiratory motion, in order to avoid motion artifacts in the image. For this purpose, it is known to obtain a pressure signal corresponding to the motion caused by respiration using a pneumatic respiratory belt. The pneumatic pressure signal is then conducted via a pressure conduit to a location remote from the belt, wherein the signal is converted into an electrical signal by a capacitive transducer or by a piezoelectric pressure sensor.

A measurement apparatus of this type is described in U.S. Pat. No. 4,324,259, wherein the pressure signal is supplied to a capacitive transducer wherein one of the capacitor plates is mounted on a flexible membrane which is deformed by the pressure signal. This causes the spacing between the plates to vary, thereby resulting in a capacitance signal which is modulated by the pressure signal. If this type of device were to be used in the environment of a magnetic resonance imaging tomography apparatus, the transducer portion of the device would necessarily have to be placed at a considerable distance from the person under examination wearing the respiratory belt, who is disposed in the magnetic resonance imaging apparatus. This is because, due to the influences of the strong high frequency fields on electronic components, it is necessary to place all electrically conductive materials at a location sufficiently remote from the fields so that the components are not affected by the fields. The necessity of using such a long pressure conduit between the belt and the transducer unavoidably results in deterioration of the pressure signal due to the inactive volume of the conduit. This decreases the sensitivity of the measuring arrangement, and may result in a faulty correlation between the derived electrical signal and the actual respiratory motion.

A respiratory motion sensor specifically designed for use in a magnetic resonance imaging environment is disclosed in U.S.4,664,129. This known arrangement includes a belt having a buckle consisting of two mating parts which are mechanically connected so as to be relatively movable. In one embodiment, a light transmitter is disposed in one of the parts, and a light receiver is disposed in the other part. Movement due to respiration will cause the light from the transmitter to be polarized by different amounts. A light polarizer is disposed in front of the light receiver. Since the light will arrive differentially polarized due to the motion. By analyzing the degree of polarization, a signal corresponding to movement of the examination subject is obtained. In another embodiment of the invention, the transmitter and receiver are disposed in the same belt part, and the other part contains a mirror which reflects the light from the transmitter back to the receiver.

US 5,088,501 discloses another respiratory belt system for being used in a magnetic resonance imaging environment. This system comprises a pneumatic respiratory belt which generates a mechanical pressure signal by means of a bellow and a pressure sensor converts the incoming mechanical pressure signal into an optical signal using a flexible membrane, which is deformed by the pressure signal, and which has a reflective surface thereon so that a modulated light signal is generated corresponding to the pressure signal. The transducer can be constructed avoiding metallic materials, thereby permitting the transducer to be disposed in the radio frequency field of a magnetic resonance imaging tomography apparatus, and thus in the immediate proximity of the respiratory belt. The pressure signals from the belt, since they must travel only a relatively short distance to the transducer, do not significantly deteriorate and thus the sensitivity of the measuring arrangement is increased.

By the above described respiratory belt systems the force by which the belt is stretched (tensioned) is converted either directly (US 4,664,129) or via a pneumatic pressure (US 5,088,501 and U.S.4,324,259) into a measurement signal. Thus the signals correspond to the force applied to the belt and not the change of girth of the person under examination. However, a signal corresponding to the amount of the change of the girth would be preferred for triggering the magnetic resonance imaging process.

Furthermore, pneumatic pressure systems are difficult to calibrate and susceptible to external conditions like accidental compression of kinking of the tube connecting the pressure sensor with the bellow.

An object of the invention is therefore to provide a respiratory belt system capable of being used in a magnetic resonance imaging environment and which provides absolute measurement signals.

The object is solved by a respiratory belt system with the features of claim 1. Advantageous respiratory belt systems are given in the subclaims.

The respiratory belt system according to the invention is suitable for being used in a magnetic resonance imaging environment. The system comprises
- a belt for being wrapped around the body of a person to be examined wherein the belt is elastically extendable,
- an optical transducer comprising a light source, an optical pattern to which light from the light source is directed and an optical sensor to which light transmitted or reflected by the optical pattern is detected,
wherein the optical pattern is coupled to the belt so that during an extension or contraction of the belt the optical pattern is moved relative to the light source for a distance being substantially proportional to the length of the extension or the contraction of the belt, so that the sensor creates a measurement signal corresponding to the change in length of the belt.

These signals provide an absolute value of the corresponding girth, if the girth is known at a particular time during the measurement period of the respiratory belt system. Such an absolute determination of girth affords the triggering of the magnetic resonance imaging process to be controlled with much greater sensitivity. Furthermore, the measurement signal of the respiratory belt system of the present invention can be calibrated to the position of an organ of the person under examination, hence, an accordingly calibrated respiratory belt system can be used for controlling the position and/or the timing of an irradiation of the organ to the position of which the respiratory belt system is calibrated. This is particularly advantageous in therapeutic appliances in which a strong irradiation is directed on a certain organ containing a tumour. As a tumour is often moved back and forth in a body due to the respiration of the person under examination, it is very advantageous if the position to which the irradiation is directed coincides with the position of the tumour either by following the movement of the tumour or by directing the irradiation only during the time when the tumour is located at a certain position.

Ordinary respiratory belt systems which create a measurement signal corresponding to the force applied to the belt are not suitable for being calibrated to the position of the organ in dependency of the girth of the person under examination.

The present invention may be better understood by taking into consideration the following detailed description of the figures and preferred embodiments of the present invention. The figures show as follows:
- Figure 1: a transducer body of the respiratory belt system according to the invention coupled to the belt in a perspective view,
- Figure 2: the transducer body of figure 1 in an explosion diagram,

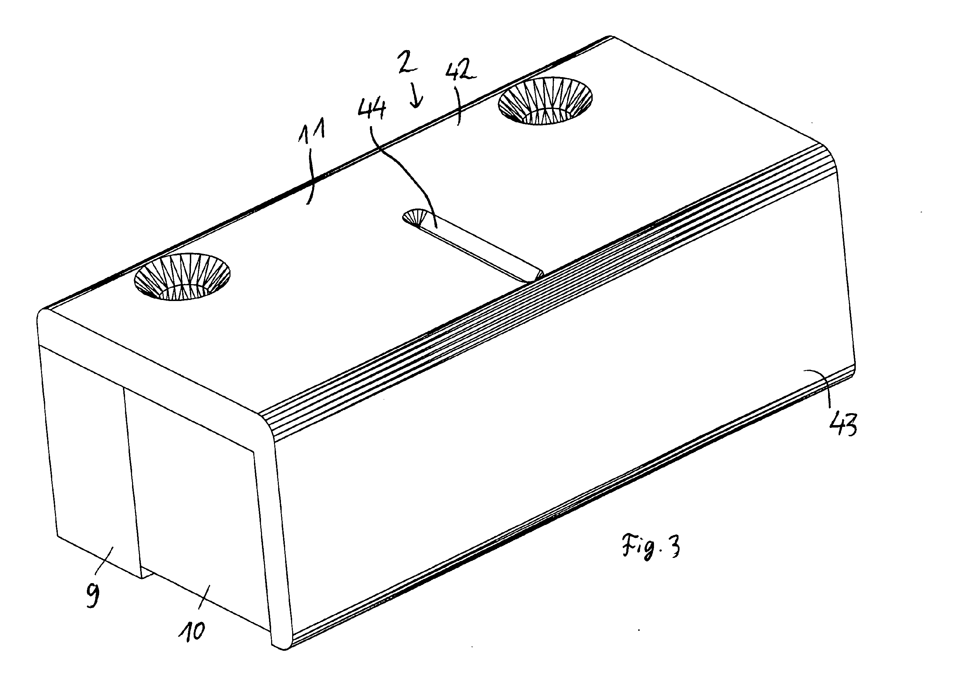

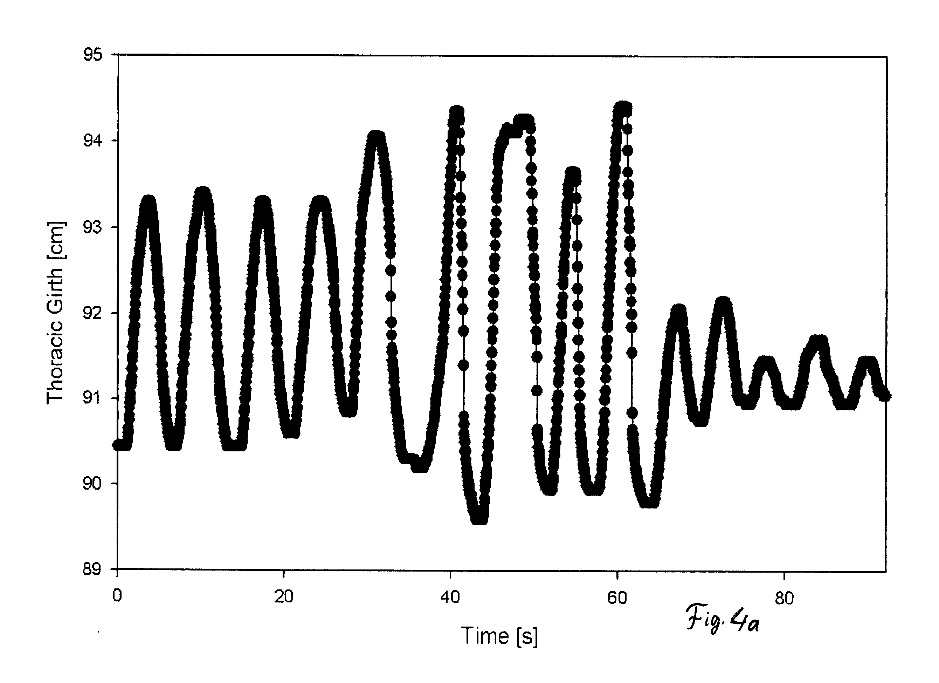

- Figure 3: the transducer body of figure 1 together with the cover,
- Figure 4a, Figure 4b: measurement diagrams of measurements achieved with the respiratory belt system according to the invention,
- Figure 5a, 5b: another embodiment of a transducer body for a respiratory belt system according to the invention,
- Figure 6: a schematical diagram of the optical signals and the direction of movement of the optical pattern, and
- Figure 7: an optical transducer having only one optical fibre for transmitting light between the light source and the transducer body and the transducer body and the optical sensor.

The respiratory belt system according to the present invention comprises a belt 1 for being wrapped around the body of a person to be examined wherein the belt is elastically extendable, an optical transducer for transforming the extension or the contraction of the belt 1 into measurement signals corresponding to the change in length of the belt.

The optical transducer according to the present embodiment of the invention comprises a transducer body 2, a light source 3, an optical sensor 4 and an optical pattern 5 being printed on an inelastic, transparent plastic strip 6.

The belt 1 comprises an inelastic section 7 and an elastic section 8. The elastic section 8 comprises two strips of elastic material like a rubber fabric. The elastic strips are connected to the same end of the inelastic section 7 of the belt 1 and are fixed with their free ends to the transducer body 2. The plastic strip 6 is disposed in between the elastic strips of the elastic section 8 and the plastic strip 6 is fixed to the same end of the inelastic section 7 of the belt 1 as the elastic strips. The elastic strips are gathered at the side edges so that they enclose the plastic strip 6. If the elastic section 8 is extended or contracted the plastic strip 6 is guided between the elastic strips like the core of a bowden cable wherein the elastic strips form the cladding.

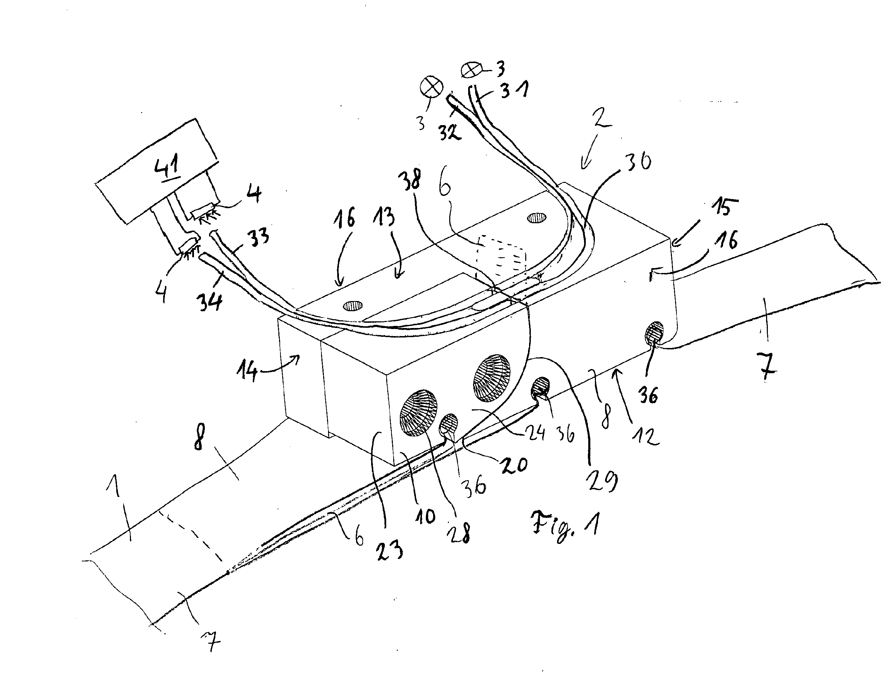

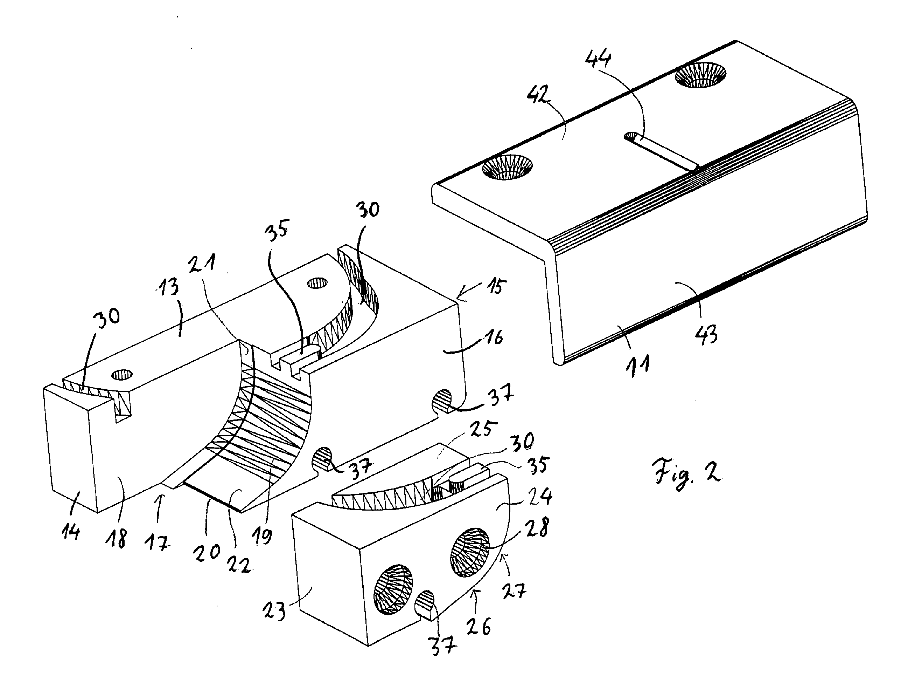

The transducer body 2 has a form of a cuboid with a length of about 60 mm, a height of about 20 mm and a width of about 30 mm. A transducer body 2 comprises three parts, a base part 9, a guiding part 10 and a lid 11 (Figure 1, 2). The lid 11 comprises an upper horizontal cover plate 42 and a vertical side plate 43. In the cover plate 42 a slit 44 is provided through which the plastic strip 6 extends.

The base part 9 has the form of a cuboid block with a lower ground surface 12 and an upper ground surface 13, a front face surface 14, a rear face surface 15 and two side surfaces 16, wherein a portion of about one quarter of the block is cut out so that a recess 17 is formed in the base part 9 having a side surface 18 being parallel to the side surfaces 16 and a face surface 19 starting in the middle of the length of the base part 9 at the upper ground surface 13 and extending vertically downwards and forming a bow in the direction to the front face surface 14, wherein this face surface 19 ends at a front edge 20. The front edge 20 is disposed at the lower ground surface 12 and the face surface 19 defines an acute angle with the lower ground surface 12.

The face surface 19 of the recess 17 has an inner strip portion 21 adjacent to the side surface 18 of the recess 17 and an outer portion 22 extending from the strip portion 21 to one of the side surfaces 16 of the transducer body 2 and this outer portion is called "lower guiding surface" 22. The width of the lower guiding surface 22 corresponds to the width of the plastic strip 6. The lower guiding surface 22 is recessed relative to the inner strip portion 21 by about 0,1 - 0,3 mm. The height of this recess corresponds about to the thickness to the plastic strip 6. The front edge 20 is rounded in the range of the lower guiding surface 22 wherein the radius is about 0,1 - 0,2 mm.

The guiding part 10 is a body having the form to be placed in the recess 17 of the base part 9 with a face surface 23, two side surfaces 24, an upper substantially planar surface 25 and a lower surface 26 having a section being formed with a bow corresponding to the bow of the lower guiding surface 22 of the base part 9. This section of the lower surface 26 which is formed in a bow is called upper guiding surface 27.

The guiding part 10 comprises two bores 28 for taking up screws and for fixing the guiding part 10 to the base part 9 in such a way that the lower surface 26 of the guiding part 10 rests on the stripe portion 21 of the face surface 19 of the recess 17. Thus, the lower guiding surface 22 and the upper guiding surface 27 are displaced by a distance corresponding to the height by which the lower guiding surface 22 is recessed relative to the strip portion 21. The lower guiding surface 22 and the upper guiding surface 27 define a slit 29 for taking up the plastic strip 6. The upper surface 25, the face surface 23 and the side surface 24 of the guiding part 10 are aligned with the upper ground surface 13, the front face surface 14 and the side surface 16, respectively.

In the upper ground surface 13 and the upper surface 25 grooves 30 are formed for taking up optical fibres 31-34. The grooves 30 are crossing the slit 29 at right angles, wherein adjacent to the slit 29 in the middle of the grooves 30 centre protrusions 35 are provided. Between the centre protrusions and the side walls of the groove the end portions of the optical fibres 31 - 34 are clamped in such a way that the face surface of an optical fibre on one side of the slit is facing the face surface of another optical fibre at the other side of the slit 29.

The remote end portions of one pair of the optical fibres 31, 32 are optically coupled to light sources 3 and the remote end portions of the other pair of the optical fibres 33, 34 are optically coupled to optical sensors 4. The length of the optical fibres should be at least 5 m so that the light sources 3 and the optical sensors 4 can be placed remote from a magnetic resonance imaging device in which the respiratory belt system is used. A length of at least 10 m, 15 m or 20 m, respectively, is preferred.

The free ends of the elastic sections 8 of the belt 1 and the inelastic section 7 of the belt 1 comprise small loops for taking up thin pins 36. These loops and pins fit into corresponding bores 37 which are open to the lower ground surface 12 of the transducer body 2 for releasable fixing the belt 1 to the transducer body 2. The belt 1 comprises a hook and loop fastener (not shown) in the inelastic section 7 for opening and closing the belt 1 and adapting the belt 1 to the girth of the person under examination.

The opposing ends of the paired fibres 31-34 define optical measuring sections 38. The plastic strip 6 is slidably guided in the slit 29 of the transducer body 2, so that the optical pattern 5 is moved back and forth through the optical measuring sections 38 when the belt 1 or the elastic section 8 of the belt 1, respectively, is extended or contracted. By the movement of the optical pattern 5 the light of the optical measuring section 38 is modulated and the modulated light is detected by the optical sensors 4 and transformed into a corresponding electrical signal.

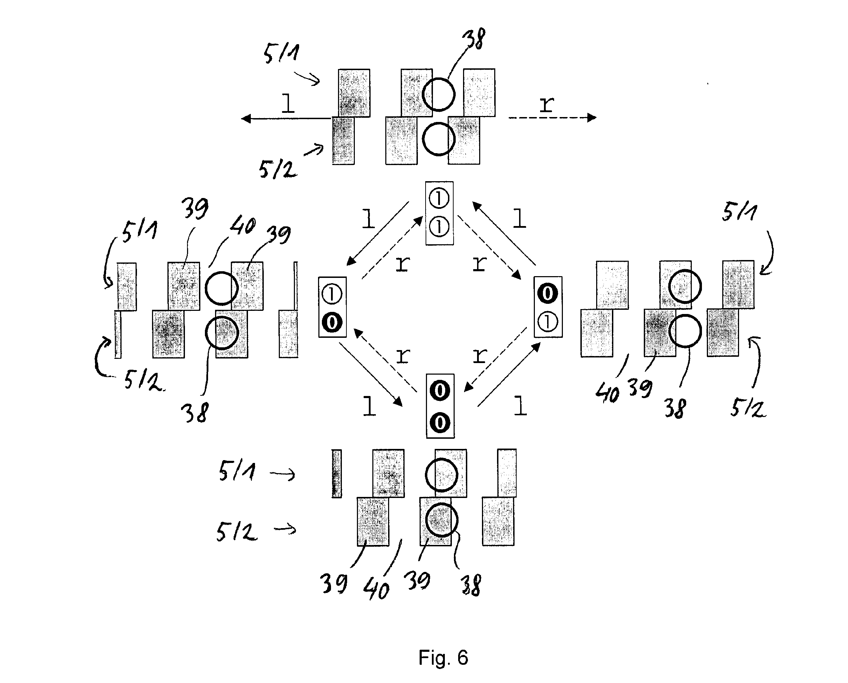

Figure 6 shows schematically a section of the optical pattern 5 relative to the optical measuring sections 38 indicated in this figure by circles. In the present embodiment of the invention for each optical measurement section 38 a separate optical pattern 5/1 and 5/2 is provided. Each pattern 5/1, 5/2 comprises alternating dark stripes 39 and light stripes 40. In the present embodiment the dark stripes are non-transparent stripes and the light stripes are transparent stripes. The widths of all dark stripes 39 and light stripes 40 are identical and equal to the diameter of the optic fibres, so that the stripes are equally displaced to each other. The provision of dark and light stripes causes a digital optical signal when the optical patterns are moved relative to the optical measuring section. When a light stripe 40 is positioned in the optical measuring section 38, the light of the corresponding light source 3 is received by the corresponding optical sensor 4 which causes the digital signal "ON" or "①", respectively. If the dark stripe 39 is positioned in the optical measuring section 38 the optical sensor 4 does not receive light from the light source 3 which causes the digital signal "OFF" or " 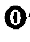 ", respectively.

The two optical patterns 5/1 and 5/2 are shifted relative to each other by the half width of a stripe 39, 40, thus it is possible that dark stripes 39 or light stripes 40 are positioned in both optical measuring sections 38 and that in one optical measuring section 38 a dark stripe and in the other optical measuring section 38 a light stripe 40 is placed and vice versa.

On the left hand side of figure 6 a state is shown, wherein a light stripe 40 of the upper optical pattern 5/1 is placed in the optical measuring section 38 and a dark stripe 39 of the lower pattern 5/2 is placed in the optical measuring section 38 which causes the signal "① 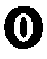 ". Moving the plastic strip 6 on which both patterns 5/1 and 5/2 are printed in the right direction moves initially a light stripe 40 of the lower pattern 5/2 into the optical measuring section 38 wherein the state of the upper pattern 5/1 does not change which results in the optical signal ①①, as it can be seen in the upper part of figure 6. If the plastic strip 6 would be moved in the left direction instead of the right direction, the signals ① 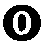 would be changed into 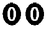 as can be seen from the lower part of figure 6. A further movement in the same direction results in the state 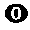 ①.

Thus by each movement of about a half width of a stripe 39, 40 only one of the two signals is changing. A transfer from ①① to ① 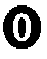 means a movement to the left direction, wherein a transfer from ①① to 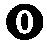 ① means a movement to the right direction. Thus, by comparing both signals which is done in an evaluation unit (not shown) the direction of the movement of the patterns 5/1, 5/2 is detected.

Generally, the detection of the direction of the movement results from a phase shift of both signals. It is not necessary to have two optical patterns. It is also possible to create such a phase shift with one single pattern and two optical measuring sections being correspondingly displaced.

The width of the stripes 39, 40 corresponds to the diameter of the optical fibres 31-34 and amounts in the present embodiment to 1,0 mm. If a higher resolution should be achieved, the width of the stripes 39, 40 can be decreased, e.g. to a width of 0,1 mm to 0,3 mm as in another embodiment. The thickness of the optical fibres 31, 32 being coupled to the light sources 3 should then be decreased accordingly or a slit diaphragm should be placed in front of the free ends of the optical fibres 31, 32 or the optical fibres 33 ,34 or both. The slit diaphragm should have a slit width corresponding to the width of the stripes 39, 40.

In use the respiratory belt 1 is wrapped around the body of a person under examination. Usually the belt is applied to the thorax or the abdomen where the girth is changing due to respiration. The change of length of the girth is measured
wherein the resolution is defined by the half width of the dark and light stripes 39, 40 of the optical pattern 5. The change of length is measured in an absolute length value. The inelastic section 7 of the belt 1 can be provided with the scale for measuring the absolute value of the girth.

The respiratory belt 1 according to the invention comprising the transducer body 2 can be used in a magnetic resonance imaging device, as it does not comprise any metallic parts and no electrical signals which could disturb or be disturbed. The light sources 3 and the optical sensors 4 are placed outside from the magnetic resonance imaging device so that the measurement is not disturbed by the magnetic field and does not disturb imaging.

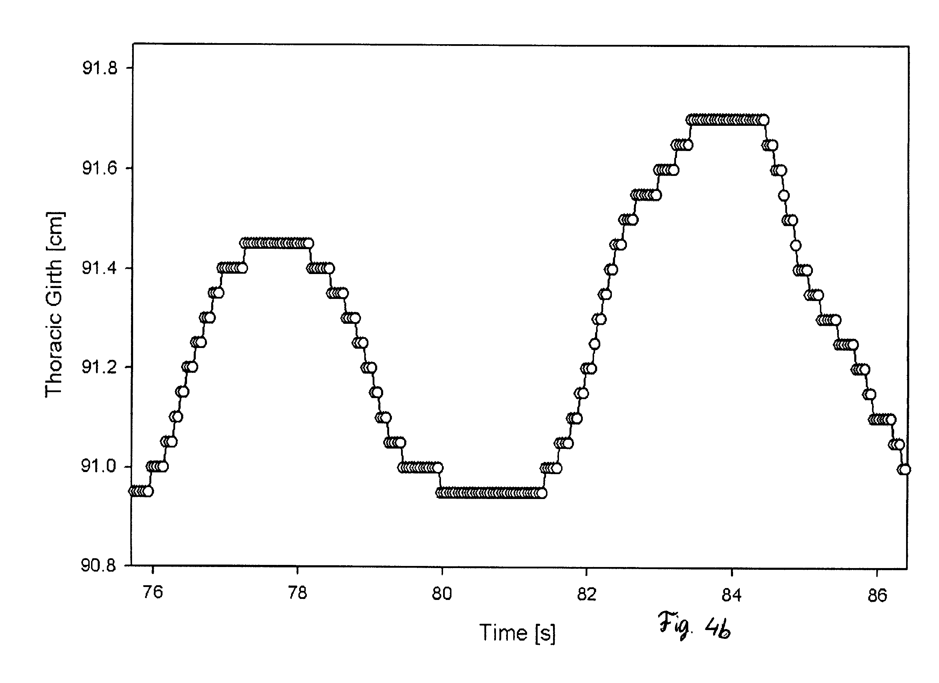

Typical measurement diagrams of measuring the thoracic girth are shown in figure 4a, 4b, wherein each measurement point is indicated by a circle (figure 4b) and the resolution of the measurement amounts to 0,5 mm.

Due to the high precision of the respiratory belt system according to the invention, it is possible to calibrate the measurement signals to the position of a certain organ, particularly of an organ containing a tumor. This calibration is done by applying the respiratory belt to a person under examination and carrying out the girth measurement and simultaneously a measurement of the position of the organ or the tumor, respectively, by a known method, e.g. computed tomography, magnetic resonance imaging, fluoroscopy or sonography. The detected position data of the organ and the corresponding measurement signals created by the respiratory belt are compared and the position data are allocated to the corresponding measurement signals. A respiratory belt system so calibrated can be used for determining the position of the respective organ or tumor, respectively, just by means of the calibrated measurement signals. This provides a very simple system for determining the position of organs moving in dependency upon respiration. The so determined position of the organ can be used for controlling irradiation to this organ. The irradiation means can be moved in such a way that the irradiation is following the movement of the organ or the irradiation can be applied to the person under test only during the time when the corresponding organ is located in the irradiation beam. Therefore, the respiratory belt system according to the invention can also form a system for controlling irradiation means by changing the direction of the irradiation or triggering the irradiation pulses in dependency upon the position of the corresponding organ. Such a system can comprise two or more respiratory belts according to the invention, wherein the signals of the belts are used for controlling the irradiation in the above described manner.

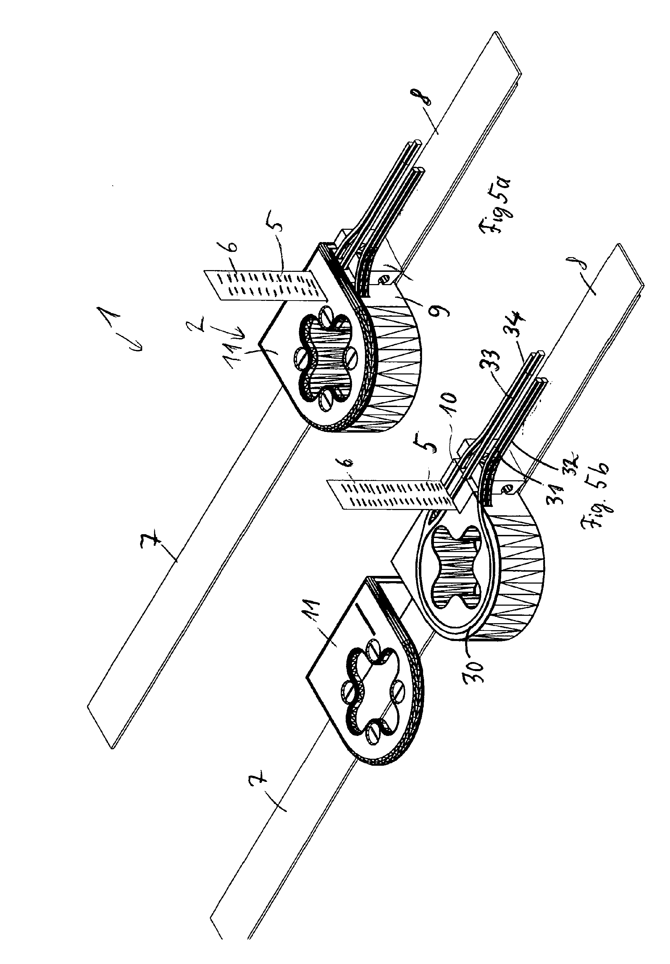

Figure 5a and 5b show another embodiment of the invention which comprises a belt 1, a transducer body 2, a plastic strip 6. The belt 1 has an elastic section 8 and an inelastic section 7. The transducer body 2 comprises a base part 9, a guiding part 10 and a lid 11. Portions of the optical fibres 31-34 are located in the transducer body 2, wherein grooves 30 for taking up the optical fibres 31-34 in the transducer body 2 form nearly a full circle so that all optical fibres 31-34 enter the transducer body 2 at adjacent positions. The mode of operation of the second embodiment is the same as that of the first embodiment.

The invention is described above by means of two embodiments having a plastic strip 6 on which the optical pattern or the optical patterns are printed. It is appreciated that the invention is not restricted to such plastic strips. The optical pattern can be provided by different means, for example it can also be printed on a non-transparent strip with a reflective surface, wherein the light source and the optical sensor are positioned at the same side of the strip. By such a device the reflected and modulated light is detected by the optical sensor. It is also possible to print a reflective material on a non-transparent but nonreflective, dark strip.

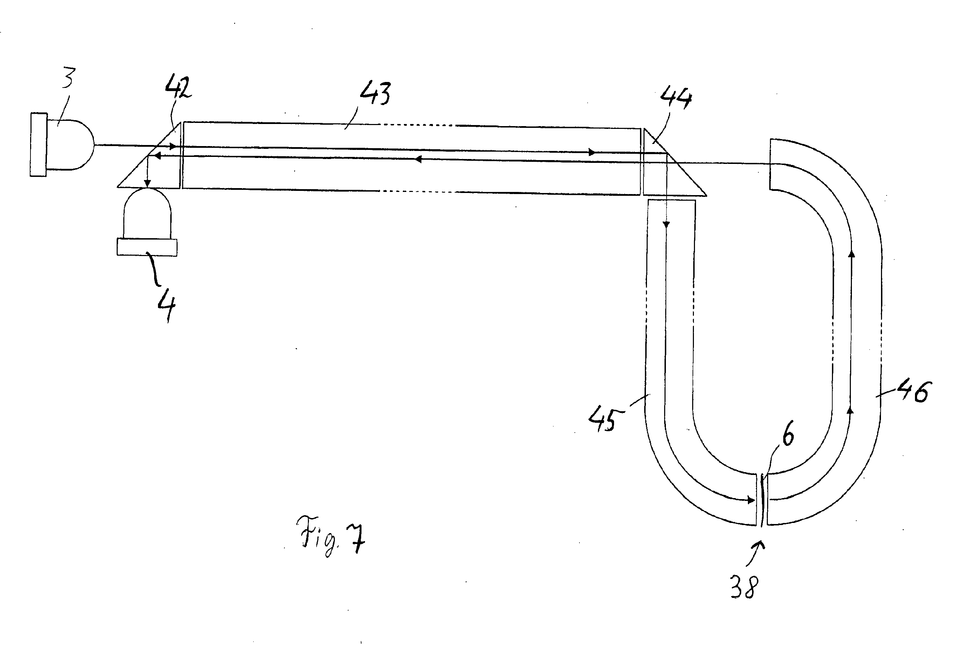

Figure 7 shows schematically an optical transducer having a light source 3, a first prism 42, a long fibre 43, a second prism 44 and two short fibres 45, 46 and an optical sensor 4.

Each prism 42, 44 has a hypotenuse and two legs. The prisms are positioned with one leg adjacent to the face surfaces of the two ends of the long optical fibre 43.

The light source 3 is positioned in line with the long optical fibre 43 so that the light is directed from the light source 3 across the hypotenuse and one leg of the first prism 42 into the optical fibre 43.

The second prism 44 is located in the transducer body (not shown in figure 7). In the transducer body the two short sections of the optical fibres form a nearly closed loop,
wherein a small gap between the two sections defines the optical measuring section 38 in which the strip 6 is positioned.

The first short fibre 45 is positioned with its face surface adjacent to a leg of the second prism 44 so that the light coming from the optical fibre 43 is reflected by the hypotenuse of the second prism 44 in a right angle towards the face surface of the first short fibre 45. The light is then guided through the first short optical fibre 45, the optical measuring section 38, and the second short optical fibre 46. The end of the second short optical fibre 46 neighbouring to the second prism is in line with the corresponding end of the long optical fibre 43, so that the light is directed from the light source 3 through the second prism 44 crossing the hypotenuse and a leg into the optical fibre 43. The light is guided by the long optical fibre 43 to the first prism, where it is reflected in a right angle towards the optical sensor 4.

This arrangement needs for each optical transducer comprising one light source and one sensor just one optical fibre between the light source and the sensor at one end of the fibre 43 and the transducer body at the other end of the fibre 43.

In a further embodiment of the invention it is also possible that the total belt is formed by an inelastic material and in the transducer body a roller is provided on to which one end of the belt is rolled up. The roller is spring loaded in such a way that the belt can be unrolled from the roller against the force of the spring. The optical pattern can be printed on the surface of the inelastic belt. It is also possible that the optical pattern is formed on the circumference of the roller or of a wheel coupled to the roller. Such a wheel can be e.g. a gear wheel, wherein the teeth of the gear wheel form the optical pattern for modulating the light of an optical measuring section.

The basic principle of the present invention is that the light is modulated by an optical pattern, wherein the optical pattern is moved relative to an optical measuring section due to the contraction and extension of the respiratory belt. As described above, the optical pattern, the coupling of the optical pattern to the belt and the arrangement of the optical elements (light source, optical sensor) can be embodied in different forms.

The invention can be summarised according to the following:
The invention relates to a respiratory belt system for being used in a magnetic resonance imaging environment.

This system comprises a belt being elastically extendable, an optical transducer and an optical pattern being coupled to the belt during an extension or a contraction of the belt. The optical pattern is moved relative to the light source for a distance being substantially proportional to the length of the extension or the contraction of the belt. The sensor creates a measurement signal corresponding to the change in length of the belt.

Such a measurement signal is much more accurate than signals of ordinary respiratory belt systems. The whole respiratory belt system has a simple design and can be cost-effectively produced.

### Reference signs

- 1: belt
- 2: transducer body
- 3: light source
- 4: optical sensor
- 5: optical pattern
- 6: strip
- 7: inelastic section
- 8: elastic section
- 9: base part
- 10: guiding part
- 11: lid
- 12: ground surface (lower)
- 13: ground surface (upper)
- 14: front face surface
- 15: rear face surface
- 16: side surface
- 17: recess
- 18: side surface
- 19: face surface
- 20: front edge
- 21: stripe portion
- 22: lower guiding surface
- 23: face surface
- 24: side surface
- 25: upper surface
- 26: lower surface
- 27: upper guiding surface
- 28: bore
- 29: slit
- 30: groove
- 31: optical fibre
- 32: optical fibre
- 33: optical fibre
- 34: optical fibre
- 35: centre protrusion
- 36: pin
- 37: bore
- 38: optical measuring section
- 39: dark stripe
- 40: light stripe
- 41: evaluation unit
- 42: first prism
- 43: long optical fibre
- 44: second prism
- 45: first short optical fibre
- 46: second short optical fibre

## Claims

1. Respiratory belt system for use in a magnetic resonance imaging environment, having
- a belt (1) for being wrapped around the body of a person to be examined wherein the belt (1) is elastically extendable,
- an optical transducer (2, 3, 4, 5) comprising
- a light source (3),
- an optical pattern (5) to which light from the light source (3) is directed and
- an optical sensor (4) to which light transmitted or
reflected by the optical pattern (5) is detected, wherein the optical pattern (5) is coupled to the belt (1) so that during an extension or contraction of the belt (1) the optical pattern (5) is moved relative to the light source (3) for a distance being substantially proportional to the length of the extension or the contraction of the belt (1), so that the optical sensor (4) creates a measurement signal corresponding to the change of length of the belt (1).

2. Respiratory belt system according to claim 1, further comprising optical fibres (31, 32, 33, 34) for guiding light from the light source (3) to the optical pattern (5) and from the optical pattern (5) to the optical sensor (4).

3. Respiratory belt system according to claim 2, wherein the optical transducer comprises only one optical fibre (43) for guiding light between the light source (3) and the optical pattern (5) on one side and a transducer body (2) on the other side wherein the transducer body contains a measurement section in which the light is directed onto the optical pattern (5).

4. Respiratory belt system according to claim 2 or 3, wherein the optical fibres (31, 32, 33, 34, 43) have a length of at least 5 m.

5. Respiratory belt system according to one of the claims 1 to 4, wherein
the belt (1) comprises an inelastic section (7) and an elastic section (8) and the optical pattern (5) is embodied on an inelastic strip (6) which is coupled to one end of the inelastic section (7) of the belt (1) and the light source (3) is coupled to the other end of the inelastic section (7) of the belt (1).

6. Respiratory belt system according to claim 5, wherein
the inelastic strip (6) is guided in the portion between the inelastic section (7) and the optical transducer (2) in between two elastic segments of the belt which are coupled to both ends of the inelastic strip (6).

7. Respiratory belt according to claim 6, wherein
the elastic segments are connected to each other at their longitudinal edges for enclosing the inelastic strip (6).

8. Respiratory belt system according to one of the claims 1 to 7, wherein
the inelastic strip (6) is made of a transparent material and the pattern (5) is printed onto the strip (6).

9. Respiratory belt system according to claim 8, wherein
the light source (3) is placed on one side of the inelastic strip (6) and the optical sensor (4) is placed on the other side of the inelastic strip (6) for detecting the light of the optical source (3) being transmitted through the inelastic strip (6).

10. Respiratory belt system according to one of the claims 1 to 7, wherein
the inelastic strip is made of a light reflecting material and the pattern is printed onto the strip.

11. Respiratory belt system according to claim 10, wherein the optical sensor is placed on the same side of the inelastic strip as the light source for detecting the light of the optical source being reflected by the inelastic strip.

12. Respiratory belt system according to one of the claims 1 to 11, wherein the pattern comprises dark and light stripes (39, 40) so that they cause a digital optical signal when the optical pattern (5) is moved relative to the light source (3).

13. Respiratory belt system according to claim 12, wherein the stripes (39, 40) are equally displaced to each other.

14. Respiratory belt system according to any of the preceding claims, wherein two optical transducers are provided and coupled to an evaluation unit (41) for detecting the direction of movement of the pattern(s) relative to the light source (3).

15. Respiratory belt system according to claim 14, wherein the two optical transducers are arranged in such a way that their signals are phase shifted relative to each other.

16. Respiratory belt system according to claim 14 or 15, wherein for each optical transducer a separate pattern (5/1, 5/2) is provided.

17. Respiratory belt system according to claim 16, wherein the two optical patterns (5/1, 5/2) are identical and are shifted to each other by the half distance between two adjacent stripes (39, 40).

18. Respiratory belt system according to claims 14 or 15, wherein the two optical transducers are co-operating with the same optical pattern, wherein the optical transducers are shifted relative to each other by the half distance between two adjacent stripes of the pattern.

19. Respiratory belt system according to one of the claims 1 to 4, wherein
the belt is formed by an inelastic material and comprising a roller onto which one end of the belt is rolled up and the roller being spring loaded in such a way that the belt can be unrolled from the roller against the force of the spring.

20. Respiratory belt system according to claim 19, wherein the optical pattern is embodied on the surface of the belt.

21. Respiratory belt system according to claim 19 or 20, wherein
the optical pattern is formed on the circumference of the roller or of a wheel coupled to the roller.

22. Respiratory belt system according to claim 21, wherein the wheel is a gear wheel and the pattern is formed by the teeth of the gear wheel.

23. Method for determining the position of a certain organ, particularly of an organ containing a tumour, in dependency of the respiration of the person comprising the organ, comprising the steps of:
calibrating the measurement signals of the respiratory belt system by applying a respiratory belt system to the person and detecting the position of the organ by a known method and
creating corresponding position data of the organ and
simultaneously detecting the measurement signals of the respiratory belt system, and allocating the position data to the measurement signals,
applying the calibrated respiratory belt system to the person and determining the position of the organ by means of the calibrated measurement signals of the calibrated respiratory belt system.

24. Method according to claim 23, wherein a respiratory belt system according to one of the claims 1 to 21 is used.

25. Method according to claim 23 or 24, wherein an irradiation of the organ is controlled according to the position determined by means of the calibrated respiratory belt system.

26. Method according to claim 25, wherein the irradiation is temporally and/or spatially controlled.
